# EUROPEAN PATENT SPECIFICATION

(11) **EP 3 072 472 B1**
(45) Date of publication and mention of the grant of the patent: **16.08.2017**
(21) Application number: 16380011.3
(22) Date of filing: 18.03.2016
(51) Int. Cl.: A61B 90/11

(54) **STEREOTACTIC WHOLE-BODY GUIDE SYSTEM FOR PRECISELY POSITIONING SURGICAL INSTRUMENTS INSIDE THE BODY**
STEREOTAKTISCHES GANZKÖRPERFÜHRUNGSSYSTEM ZUR GENAUEN POSITIONIERUNG VON CHIRURGISCHEN INSTRUMENTEN IM KÖRPERINNEREN
SYSTÈME DE GUIDAGE DE CORPS ENTIER STÉRÉOTAXIQUE SERVANT À POSITIONNER AVEC PRÉCISION DES INSTRUMENTS CHIRURGICAUX À L'INTÉRIEUR DU CORPS

(30) Priority: 23.03.2015 ES 201500219 P
(43) Date of publication of application: 28.09.2016
(73) Proprietor: Rey Portolés, Germán Carlos, 28007 Madrid (ES); Cabrera, Yamira, 28100 Alcobendas, Madrid (ES)
(72) Inventor: Rey Portolés, Germán Carlos, 28007 Madrid (ES); Cabrera, Yamira, 28100 Alcobendas, Madrid (ES)

(56) References cited:
- WO-A1-00/30557
- US-A- 5 242 455
- US-A- 5 603 318
- US-A1- 2006 122 629
- US-A1- 2013 267 963
- US-B1- 6 350 076
- US-B1- 6 826 423

## Description

### TECHNICAL FIELD

This invention pertains to the technical sector of medical devices, more specifically to the field of minimally invasive interventional medicine, namely, devices that guide a minimally invasive surgical instrument (the tip of a rigid needle or drill bit) inside the trunk of a human being or animal in order to perform localized procedures with precision in a well-defined area of the body, for instance, biopsies.

### BACKGROUND OF THE INVENTION

The object of the invention is a medical apparatus for use on a living being (human or animal) in order to be able to reach structures located inside the body. This is carried out by means of straight, minimally invasive instruments such that, when the target has been reached, a tissue sample may be removed, a substance delivered, fluid extracted, ablation performed, etc.

In surgery, stereotactic, or stereotaxic, is used to describe a procedure that makes it possible to access a target, generally one that is not visible, by means of a navigation system that makes use of a reference system which is itself mapped onto the subject on whom the surgery is performed. The purpose is to place a physical device, e.g., the tip of a needle, within the target or to focus ionizing or sonic radiation on that point.

Intracranial stereotactic neurosurgery is a well-established procedure that makes it possible to reach internal brain structures using precision instruments for a variety of purposes, chief among which are:
- Extracting samples of a mass that is pathological in appearance (biopsy).
- Implanting electrodes in order to analyse electrical signals in the brain and/or produce a temporary stimulation or alteration in function.
- Implanting electrodes to cause a definitive lesion (ablation), generally using radiofrequency to raise local temperature.
- Extracting fluid from a cyst pressing on the brain parenchyma.
- Introducing a chemical substance (a medication) to try to improve a patient's condition.
- Making extremely precise perforations in a bone using a drill bit.

There are a number of devices for performing procedures of this kind on the head. The first to be used on the human head were developed between 1947 and 1949 by the neurologist Ernest A. Spiegel and the neurosurgeons Henry T. Wycis and Lars Leksell.

These procedures continue to be in use today, aided by ongoing advances in medical imaging and computer science. This is underpinned by two attributes that considerably simplify procedures by allowing stereotactic navigation to any point within the brain: firstly, the presence of a rigid cranial vault to which a stationary reference frame may be fastened by screws, following which a mechanical apparatus bearing a medical instrument may be attached to the frame; and secondly, the fact that for all practical purposes the brain structures located within the skull do not move.

Since 1950 a number of inventors have patented a range of devices of different kinds. However, at present the device developed by Leksell and sold by the Swedish company Elekta AB (Leksell Stereotactic System®) has a market share of practically 80 %. The images that enable the target to be visualized are taken with the frame in place, and concomitantly it is also necessary to visualize a series of fiducial marks that serve to pinpoint the location of the target within coordinates relative to the frame.

Equipment that does not require anchoring the stereotactic frame to the head began to appear towards the end of the twentieth century. Still, the head has to be held in place using screws in the operating theatre, and the images of the patient taken previously have to be correlated (coregistered) with points on the bone structure located by means of pointers. These procedures are known as "frameless" procedures.

The problem arises when it is necessary to reach a target outside the head. There is no simple way to hold the human body firmly still, as there is for the head, and what is more it is known that the inside of the body may move in relation to any rigid (bone) structure that one might try to use as a reference point for purposes of target location.

The simplest solution has been to take a series of images and then, without changing patient position, aim the instrument employed towards the desired point in the body using the platform on which the patient is lying. Attaining the level of precision that can be achieved inside the skull is unquestionably much more complicated, though this is offset by the greater margin of error that can be tolerated outside the brain.

The procedure generally employed is wholly manual, consisting of taking measurements, on the monitor (console) of the imaging equipment, of the position and angle of entry in the plane of the image on the screen and inserting a needle little by little, using a series of scans of the patient to check that the correct path is being followed. If not, the needle has to be withdrawn and a fresh attempt made along a new path. It can be readily intuited that this procedure is subject to numerous shortcomings and considerable imprecision, and generally it is not used for small, deep-lying targets. Another major drawback to the manual system is that it takes a long time and greatly increases both the effective dose received by the patient and exposure of health care professionals to radiation (where an imaging modality employing ionizing radiation is used).

Patents have been filed for some inventions that have sought to mitigate these shortcomings and allow specific parts of the body to be reached with a given degree of precision. Most are intended to reach a mass within the female breast in order to remove a tissue sample for analysis (biopsy), using magnetic resonance imaging. There are also a number of devices that are intended for reaching the prostate for that same purpose and even for therapeutic purposes. In the case of the prostate ultrasound is ordinarily the imaging modality employed. Lastly, many special procedures have as their target the spinal column, but because the devices that have been developed for this are closely reliant on a bony structure, they are generally not transposable to other parts of the body.

Very few devices have been invented for the purpose of reaching anywhere within the body, especially the torso, the object of the invention described here. These can be divided into two categories:
- The first category is a computer controlled manipulator that holds the needle and places it in position for the health care professional to exert pressure to insert the needle to the point indicated by the manipulator. The following patents and patent applications are examples of this category: US 9546279 B1, which has the added particularity of being able to move the patient and subsequently reposition the patient with the aid of fiducial markings; WO 97/42898; KR 10-2011-0069206; US 5078140, which is also intended for use on the brain; US 6245028 B1; and US 2014/0316259 A1.
- The second category is a mechanical device that provides for simple manual alignment of a needle with the aid of computer software.

The invention described here is a device of the second type. This group would also include devices that are placed on the patient's skin and guided by means of arcs, such as WO 2008/047379 A2, WO 2008/062474 A2, US 6689142 B1, and US 2014/0336670 A1.

The following patents have as their object a needle holder that is placed within a structure around the patient, whereby they provide greater stability than the ones referred to above: JP 390041542, whose design is more suitable for the head and differs substantially from the object of this invention; US 5308352 and US 5665095, which use graduated semicircles to direct the needle and determine the corresponding angle of entry; and CN 203252733 U, which is very similar to the ones just mentioned but uses different fiducials to relate the structure to the body of the patient.

At the present time the only alternatives to the object of the invention presented here are manual or manipulators that have some servo elements (but without haptic perception safety systems and hence capable of causing injury when the patient moves inadvertently). A recent publication is cited here by way of example:
"Design and Kinematic Analysis of 3PSS-1S Wrist for Needle Insertion Guidance", Lisandro J. Puglisi, Roque Saltaren, German Rey Portoles, Hector A. Moreno, Pedro F. Cardenas, Cecilia Garcia. Robotics and Autonomous Systems. Volume 61, Issue 5, May 2013, Pages 417-427.

US5242455 describes a stereotactic localization device for use in conjunction with an imaging system to place a probe within a human body having a base plate with reference markers, a bridge, fastening means for ensuring immobility and reproducibility of the position of the bridge with respect to base plate and a frame placed on top of the bridge. WO0030557 describes a manipulator having a frame with two stationary guide members parallel to each other and to the base plate, two carriages, one of the two carriages being located on each of the two stationary guide members and being able to slide along the respective stationary guide member in a direction perpendicular to the long axis of the base plate, wherein each carriage is carrying another inverted carriage through which a movable guide arm is able to slide in a direction perpendicular to the direction of motion of the carriage such that its movement is parallel to the long axis of the base plate, allowing the two movable guide arms to move freely in two parallel planes and parallel to the base plate. US2013267963 describes a ball joint with a through hole for positioning a needle. The present invention not only allows haptic perception providing for variable attachment of the needle to the device but also combines this with manual guidance, with the resulting cost savings and savings in the calibration time required by electromechanical devices of any kind prior to practical use. It is clear that the invention could also incorporate measuring means for positioning the movable members as well as servomechanisms capable of readily placing the movable members in the proper position for needle insertion. These means will be the subject matter of future patents once the manual device described here has demonstrated its efficacy and safety in clinical practice.

### EXPLANATION OF THEINVENTION

The stereotactic whole-body guide for precisely positioning surgical instruments inside the body of this invention is a device that has a base plate with reference markers (hereinafter fiducials). The base plate is fastened to the examination table of the imaging system [for example, a CAT (computed axial tomography) table]. The patient is immobilized on the base plate using any standard procedure (a form-fitting mattress, straps, wedges, etc.), the degree of immobility depending on the degree of precision sought. After an imaging test has been performed, the patient is kept in position on the base plate, either on the CAT table itself or on a table capable of supporting the base plate without the patient moving or getting up.

A rigid structure, i.e., a bridge, is placed on the base plate around the patient. The fastening means should ensure that the bridge does not move and that the bridge can be replaced on the base plate in the same position.

A structure, i.e., a frame, composed of two parallel guide members, is placed on tne cross-piece of the bridge if the needle is to be inserted from above, or on the side pieces of the bridge if the needle is to be inserted through the patient's side. A carriage is then attached to each guide member such that it can slide along the guide member perpendicularly to the long axis of the base plate. In its turn, each carriage bears attached a second inverted carriage through which a second movable guide arm can slide perpendicularly to the above-mentioned guide member, i.e., in a direction parallel to the long axis of the base plate.

Spherical supports are attached to the tips of each of the movable guide arms. The position of the two spherical supports in space is determined by the position of the carriages on the guide members perpendicular to the long axis of the base plate and the positions of the movable guide arms parallel to that same axis.

The support system comprises the tip at one end of the guide arms, to which the spherical supports are attached. Attachment may be achieved by magnetic force or by suction. Whichever method is used, the spherical supports should be capable of rotating freely such that a hypothetical line passing through the centres of the spherical supports will be able to define any needle path. Passing through each of the spherical supports there is a straight through hole drilled through the centre.

Inserting a needle or any straight device having a diameter similar to the size of the through hole and attaching the spherical support and needle assemblies to the tips of the movable guide arms thus automatically aligns the needle along the line defined by the positions of the carriages and the movable guide arms.

In addition, the images of the interior of the patient's body, on being visualized in standard DICOM format, are transmitted to a special computer program capable of simulating paths followed by the needle and determining the point of entry of the needle through the skin and the angle of entry needed to reach a given target at depth. This software locates the fiducials. The fiducials define a reference system embedded in the base plate and hence part of the structure formed by the guide elements. The software is capable of defining any path in terms of parameter values linked to the position of the guide elements on the device (readily determined by means of rules affixed thereon). Attaching the spherical supports in which the needle or needle alignment system has previously been inserted is all that is needed to align the needle in the direction for entry into the body. The software returns the number of millimetres the needle needs to traverse. To ensure that this depth is not exceeded, a marker or a mechanical stop is affixed to the needle or the needle aligner.

If the patient remains on the same imaging system table until the end of the insertion, the needle position can be monitored during the insertion. This is not possible if the patient is placed on a transport stretcher and moved out of the room on the base plate.

The subject matter of the invention falls in the field of minimally invasive interventional medicine. A specific target inside the body is visualized on a medical image of the patient, such as a CAT (computed axial tomography) or an MRI (magnetic resonance imaging) scan, and a safe path to reach that target from outside the body is plotted and indicates how to align the medical instrument, such as a rigid needle, drill bit, etc., for insertion into the body to the intended target by the operator.

This is a medical procedure intended for use on a living being in order to reach structures located inside the body using a minimally invasive, straight instrument, such that, once the target has been reached, a tissue sample or fluid may be removed, a substance delivered, ablation performed, and so forth.

Using the device described by this invention requires:
a) Medical imaging equipment such as an axial tomography scanner, a magnetic resonance imaging scanner, or other imaging modality equipment.
b) The guidance system capable of positioning a needle in the proper position over the skin, the subject matter of the invention.
c) A computer software algorithm that determines the position of the guidance system and the depth to be attained. This requires locating fiducials and defining a coordinate system associated with those fiducials.
d) Insertion of the needle to the target identified by the imaging system by an operator, who then performs the intended action.

One of the main advantages of this procedure is that it ensures that the patient remains immobile during the entire procedure. To this end supplementary laser diode-based means have also been developed for placement on the patient. The laser beam is projected onto a radio-transparent screen attached to the base plate by fastening means, likewise radio-transparent. A video camera is affixed to the laser diode to monitor beam position on the screen. The screen may be bent to a certain extent to be able to fit into the tunnel of the CAT or MRI scanner, and it is provided with multiple orifices into which markers (small plastic pegs) can be readily inserted from outside (the side opposite the laser dot) to indicate where the beam hits before the procedure. The procedure may then proceed to conclusion, keeping the laser beam focused on the marker. The challenge that is faced is that the target moves as the patient breathes. This requires placement of at least two markers of beam position, one on inhaling and the other on exhaling. The markers also serve as a position monitoring aid when the patient is asked to shift position for the CAT scan or on insertion of the needle.

The device of the invention affords the following advantages:
1. Thanks to means developed as part of the invention, the spherical supports are displaceable in two parallel planes. This system simplifies the guidance mechanism while minimizing mechanical positioning errors.
2. The position of the spherical supports is determined by image analysis of the images of the patient that contain fiducials which make it possible to ascertain device position as it relates to the patient and thereby to suitably position the spherical supports.
3. The device enables the gripping force of the spherical supports to be adjusted, thereby lessening the risks attaching to abrupt patient movement after the needle has been inserted.
4. The device considerably reduces the amount of time needed to perform manual interventional radiology insertion procedures. This results in better use of imaging equipment, lowers the effective dose received by the patient (where ionizing radiation is employed), and enhances patient comfort by decreasing the number of punctures required to reach the target.
5. The device enables continuous monitoring of patient position while CAT images are being taken and during puncture, thereby ensuring that no untoward movement has occurred and that breathing movements can be monitored as necessary.
6. The device allows easy, safe, and quick access to small internal lesions in places that cannot be accessed by conventional procedures due to the risk of missing the target or causing injury to a vital organ in the vicinity of the lesion.

### BRIEF DESCRIPTION OF THE DRAWINGS

Figure 1 is a perspective view of the stereotactic guidance device for precisely positioning surgical instruments inside the body. The frame has been placed on the beam of the bridge.
Figure 2 is a view of one of the preferred embodiments of the invention showing a needle pushed by means of an extender.
Figure 3 is a detail view of the tips of a guide arm to which the spherical supports are attached by means of a magnet.
Figure 4 is a schematic of an auxiliary device, an extender, for aligning the needle when the needle is very flexible or long.
Figure 5 is a detail view of the two carriages that are assembled together in order to move the tip of the movable guide arm through different points in the plane defined by the stationary guide members.
Figure 6 shows the arrangement of the laser diode on the patient to monitor laser beam position during the procedure.

### PREFERRED EMBODIMENT OF THE INVENTION

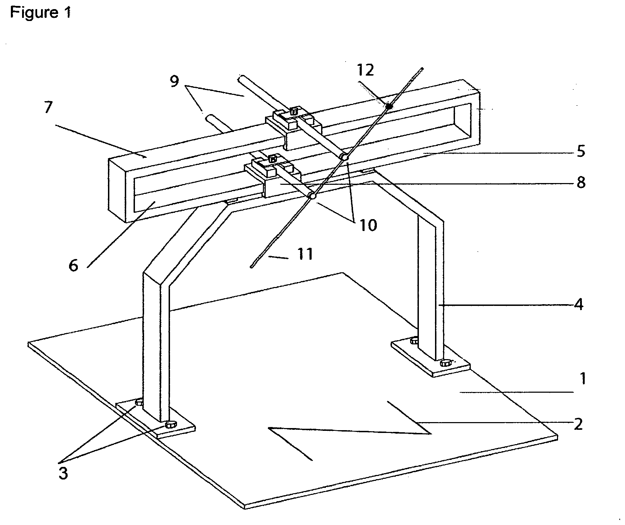

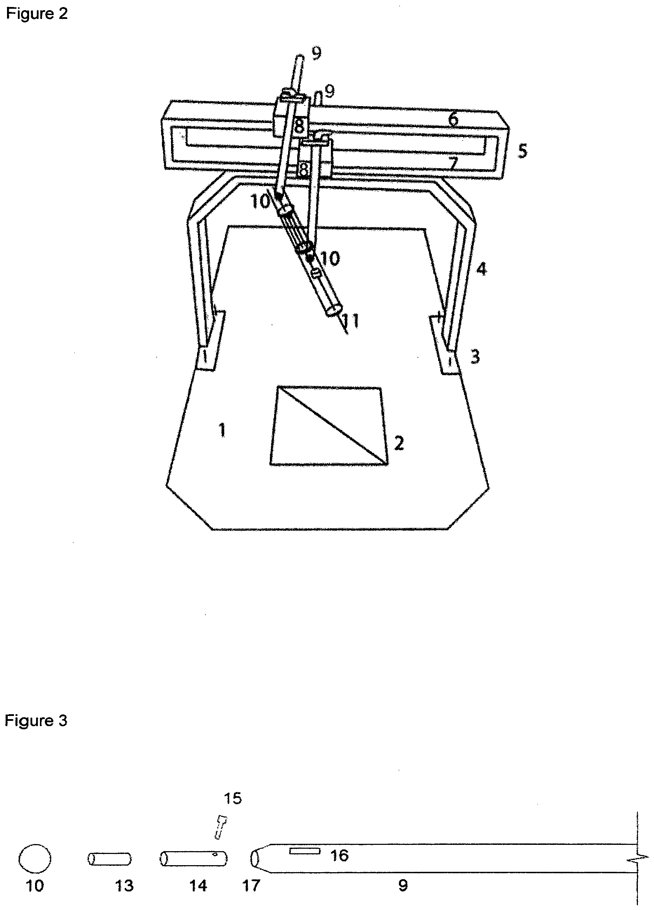

Figure 1 is a perspective view of the stereotactic whole-body guidance device for precisely positioning surgical instruments inside the body. Figure 2 depicts an example of a preferred embodiment of the invention of the stereotactic whole-body guide system. The device is erected on a base plate (1) made of a radio-transparent plastic material 6-mm thick, 600-mm wide, and 1 000-mm long. The base plate (1) is placed between the patient and the table of a CAT scanner or other similar imaging system.

Patient position on base plate (1) should remain unchanged throughout the entire duration of the procedure.

Base plate (1) is characterized by having within its underside a groove (2) for housing fiducials, the groove taking the form of a square with a side length of 200 mm plus one of the diagonals of the square. A copper wire 1 mm in diameter (for CAT scans) or tubing containing a 5 per 1 000 copper sulphate solution (for MR imaging) is embedded within groove (2). In this way, when tomography of the patient is performed, slices of the patient's internal structures can be viewed in association with a slice of the square with its diagonal (2), thereby enabling the position of base plate (1) to be mapped onto any internal structure within the patient.
Accordingly, this provides Cartesian coordinates for any point within the body in terms of the coordinate system, i.e., "the stereotactic coordinates", defined by the square (2) embedded in base plate (1). Base plate (1) is provided with four lugs (3). After the scan has been taken, without moving the patient, a structure, i.e., a bridge (4), is set on base plate (1) by means of lugs (3); bridge (4) is made of steel and measures 530 mm in width by 400 mm in height. It should be fabricated so as to achieve an extremely high degree of rigidity. The upper section of bridge (4) needs to be meticulously parallel to base plate (1), i.e., having an i inclination of less than 1 mm/m.

Frame (5), having two stationary guide members (6) and (7), is placed on steel bridge (4), on top or fastened to either of its side pieces, depending on the area of the body in which the surgical instrument is to be inserted (front, left side, or right side); the Figure shows frame (5) placed on the top of bridge (4). The two stationary guide members (6,7) are 600 mm long and are joined together at each end by a rectangular section 80 mm high by 80 mm wide. Stationary guide members (6,7) are engraved with a millimetre rule. Stationary guide members (6,7) define the planes in which spherical supports (10) move. A carriage (8), an L-shaped steel section, is slidingly mounted on each of stationary guide members (6,7) on frame (5) and is capable of being precisely positioned using the millimetre rule on each guide member (6,7); carriages (8) are secured in place by tightening a screw clamp (34). There is one carriage (8) on each stationary guide member (6,7). Affixed on top of each carriage (8) there is a second, inverted carriage (33) through which a movable guide arm (9) made from a hollow steel rod is slidingly mounted. Each movable guide arm (9) moves perpendicularly to the respective stationary guide member (6,7), and its position relative to the said stationary guide member (6,7) can be determined by means of a millimetre rule. The movable guide arms (9) are secured in place on each carriage (8) by tightening a screw clamp (32).

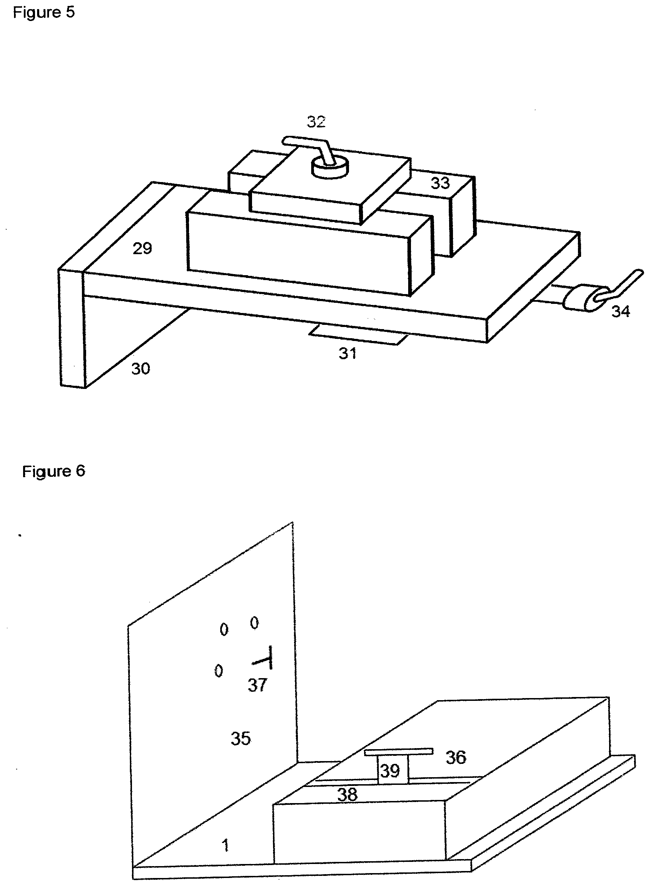

Figure 5 is a detail view of a carriage (8), made of a steel cuboid (29) that rests on one of the stationary guide members (6,7), another steel cuboid (30) that serves to align carriage (8) on the respective stationary guide member (6,7), a plastic tab (31) for easily reading the position of carriage (8) along the millimetre rule on the respective stationary guide member (6,7), and a screw clamp (34). In addition, mounted on the top of each carriage (8) there is an inverted carriage (33) through each of which a movable guide arm (9) slides. Each movable guide arm (9) is a hollow steel cylinder 12.5 mm in diameter and 600 mm long.

The position of movable guide arm (9) through each inverted carriage (33) is measured by means of a ruler or a millimetre rule engraved on the guide arm (9) itself. A screw clamp (32) helps to secure the movable guide arm (9) in the appropriate position.

Figure 3 shows a magnet (13) that serves as means for attaching the spherical supports (10). One end of each movable guide arm (9) tapers to a tip having the shape of a truncated cone (17), narrowing the inside diameter of the hollow arm to 7 mm. Figure 3 also depicts a detail of this end of movable guide arm (9). The said tip of hollow movable guide arm (9) houses the said magnet (13), which is 5 mm in diameter by 15 mm in length, cylindrical in shape, and made of neodymium (NdFeB), and the magnet is, in its turn, inserted into a hollow steel cylinder (14) having an inner diameter of 5 mm, an outer diameter of 7 mm, and a length of 20 mm and also having a through hole for a screw (15) at the rear. The cylinder and magnet assembly is inserted into movable guide arm (9) through the tapered tip (17), and its position inside is adjustable to be closer to or further away from the tip (17) of each movable guide arm (9) by means of a slot (16) in movable guide arm (9) and screw (15), whereby the attractive force of magnet (13) relative to spherical supports (10) placed against the tip (17) of each movable guide arm (9) can be adjusted until it is practically nil.

The two spherical supports (10), each measuring 12.5 mm in diameter and having a hole passing through the centre that is between 1 and 2 mm in diameter for a tight fit to the calibre of the needle (11) or extender (27) as called for by patient anatomy or needle length are situated at a given location within the two planes defined by carriage (8) on stationary guide members (6,7) on frame (5) and by the position of each of the movable guide arms (9) relative to inverted carriages (33).

Spherical supports (10) are threaded onto needle (11) or extender (27) (depending on needle size) and then placed on the tips of the two movable guide arms (9) by hand. This automatically aligns needle (11) along the projected path. The drawings depict a commercially available needle (11) and an extender (27), onto which the spherical supports (10) and a coupler (28) which connects the extender to the head of the needle, have been threaded (through the 2-mm through hole), coupler (28) being designed according to needle type.

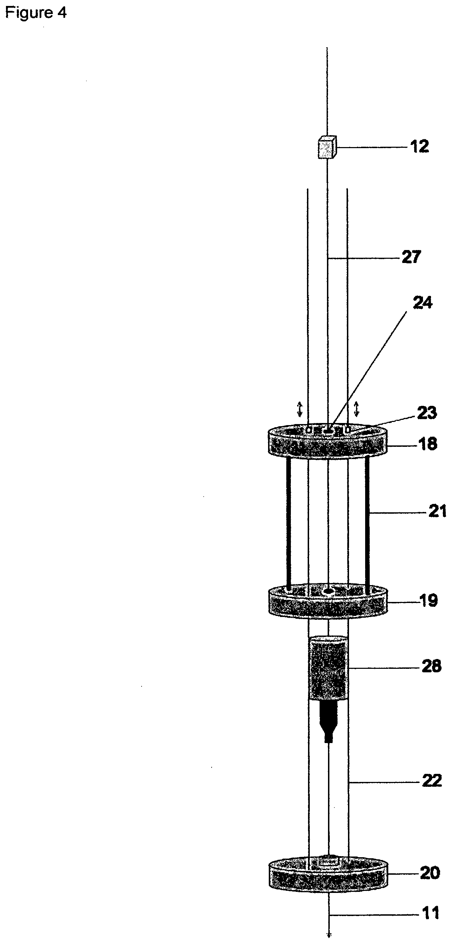

To mark the needle length to be inserted into the body of the patient to reach the target point, a stop (12) is placed on extender (27). In the case of very long needles, extender (27) may be dispensed with, and stop (12) may be placed directly on the needle itself. Stop (12) is depicted in Figure 4.

Inasmuch as the needles used for these purposes are extremely long and narrow, generally less than 1 mm in diameter, they are commonly quite prone to flexing. To ensure that the tip of the flexible needle is aligned with the extender, needle alignment means are employed as shown in Figure 4, the said means being formed by three discs (18,19,20) 30 mm in diameter and 5 mm thick, made from a light-weight material such as Teflon®. Discs (18) and (19) are held in alignment by two rigid carbon fibre rods (21) 2 mm in diameter by 60 mm in length. The third disc (20), having similar features, is provided with two thin carbon fibre rods (22) 2 mm in diameter by 300 mm in length capable of sliding through orifices in the other two Teflon® discs (18,19) as depicted in Figure 4.

The two discs (18,19) have a through hole (24) 2 mm in diameter in the centre, while the third disc (20) has a 1-mm through hole.

Needle (11) or needle extender (27) is inserted not only through the two metal spherical supports (10) but also through the three discs (18,19,20). The relative positions of the spherical supports (10) and discs (18,19) will depend on needle length and the distance of the patient from the spherical supports (10). The third disc (20), which as described is movable with respect to the other two discs (18,19), is held parallel to the said two discs (18,19) by means of the narrow rods (22). The third disc (20) is placed near the tip of needle (11) as close as possible to the skin without touching the skin to ensure that the device does not change direction, and to hold it in that position each of the two narrow rods (22) has an adjustable retainer (23). Once the needle has entered the skin, disc (20) may be allowed to touch the skin, because the needle will no longer be able to deviate from its path, and if necessary one of the spherical supports (10) may be detached by moving magnet (13) to release it if either of discs (18,19) interferes with it.

The system for monitoring the patient's breathing comprises a screen (35) made of a radio-transparent material like that of the thermoplastic masks used to immobilize the heads of patients during radiotherapy and is depicted in Figure 6. Screen (35) is 300 x 230 mm in size and is adjustable so that it may be positioned appropriately with respect to patient anatomy and as may be required to pass through the tunnel of the CAT scanner by being bent by means of a hot water bath. Screen (35) is attached to base plate (1) by means of a carbon fibre right-angle section. Attachment position is adjustable for best placement with respect to the point of puncture by means of insertion into holes made in base plate (1). Additionally, it is advantageously perforated with 1.5 mm orifices. The orifices are suitable for inserting, from the side opposite the laser beam, a peg or the like made of copper or other material that does not cause CAT artefacts but is visible when struck by the laser beam. A box (36) measuring 50 x 50 x 30 mm having a laser diode is positioned with the aid of a Velcro strip (38) that attaches to another Velcro strip around the patient. When the laser is turned on, the dot on the screen produced by the laser beam is directly visible, and a marker (37) may be put in place with the aid of the perforation pattern.

The laser diode should not be located in the region where the CAT scan is to be performed, but if the procedure is to be undertaken in a location subject to respiratory movement, it should be placed at a spot on the patient's skin that moves with breathing so that movement of the dot on the screen produced by the laser beam can be observed. In such cases at least two markers will be inserted in the path of the laser beam, at the end points for inhaling and exhaling.

In order to be able to monitor the procedure even when the patient is inside the CAT scanner, it is appropriate to attach a small USB camera (39) to the laser, connected to a computer by means of a cable. By this means it is possible to ensure that the patient is in the position required by the physician before exposure to ionizing radiation and that the position can be held as required.

## Claims

1. Stereotactic whole-body guide system for precisely positioning surgical instruments inside the body, comprising:
- a base plate (1) having fiducials (2)
- a bridge (4)
- fastening means (3) that ensure immobility and reproducibility of the position of the bridge (4) with respect to the base plate (1)
- a frame (5) placed on top of or on one of the side pieces of the bridge (4), **characterized by**
- the frame (5) having two stationary guide members (6,7) parallel to each other and to the base plate (1);
- two carriages (8), one of the two carriages (8) being located on each one of the two stationary guide members (6,7) and being able to slide along the respective stationary guide member (6,7) in a direction perpendicular to the long axis of the base plate (1), wherein each carriage (8) is carrying another inverted carriage through which a movable guide arm (9) is able to slide in a direction perpendicular to the direction of motion of the carriage (8) such that its movement is parallel to the long axis of the base plate (1), allowing the two movable guide arms (6,7) to move freely in two parallel planes and parallel to the base plate (1); and
- a system comprising spherical supports (10) that uses magnetic force or suction to attach the spherical supports (10) to the movable guide arms (9) and is capable of producing differing intensities in the force of attachment between the spherical supports (10) and the movable guide arms (9), wherein the spherical supports (10) are able to rotate freely, such that a hypothetical line passing through the centres of the spherical supports (10) can define any path that may be followed by a needle (11), wherein each spherical support (10) has a straight through hole passing through its centre.

2. Stereotactic whole-body guide system according to claim 1, **characterized in that** the spherical supports (10) are attached to one of the ends of each of the movable guide arms (9), wherein the position of each spherical support (10) in space is thus determined by the position of the two carriages (8) on the stationary guide members (6,7) perpendicular to the long axis of the base plate (1) and by the position of the movable guide arms (9) parallel to that same axis.

3. Stereotactic whole-body guide system according to claim 1, **characterized in that** it has a system of fiducials (2) for correspondingly mapping the coordinate system of a scanner onto the coordinate system of the stereotactic whole-body guide system, the said system of fiducials being composed of a geometric shape formed by material visible to the scanner embedded within the base plate (1) connected to the stereotactic whole-body guide system and placed on the table on which the patient reclines.

4. Stereotactic whole-body guide system according to claim 1, **characterized in that** it has a system for aligning flexible needles composed of three discs (18,19,20) made of a light-weight material, the first two discs (18,19) being connected by rigid rods (21) that hold them parallel to each other, whereas the third disc (20) has two long rods (22) that pass through two through holes in the other two discs (18,19), wherein the rods (22) keep the third disc (20) parallel to the other two discs (18,19) irrespective of the distance separating them, wherein the three discs (18,19,20) have an orifice (24) in the centre for a needle (11) and a needle extender (27) to pass through and the needle (11) or needle extender (27) traverses the three discs (18,19,20) and the two spherical supports (10), wherein the spherical supports (10) hold the two interconnected discs (18,19) in the proper orientation, while the third disc (20) is brought close to the patient's skin so as to properly guide the tip of needle (11).

5. Stereotactic whole-body guide system according to claim 1, **characterized in that** it has a system for monitoring patient movement during the procedure composed of a laser diode (36) that shines its beam onto a radio-transparent screen (35) attached to the base plate (1) of the stereotactic whole-body guide system, the said screen (35) having means (37) for marking the position of the beam and of a USB camera (39) attached to the laser diode used for monitoring, at a distance, to ensure that the laser beam hits the marker.

## Patentansprüche

1. Stereotaktisches Ganzkörperführungssystem zur präzisen Positionierung von chirurgischen Instrumenten im Inneren des Körpers, **dadurch gekennzeichnet, dass** es umfasst:
- eine Basisplatte (1) mit Bezugszeichen (2)
Eine Brücke (4)
- Befestigungsmittel (3), die die Beweglichkeit und Reproduzierbarkeit der Lage der Brücke (4) gegenüber der Grundplatte (1) gewährleisten,
- einen Rahmen (5), der oben oder auf einem der Seitenteile der Brücke (4) angeordnet ist, **dadurch gekennzeichnet, daß** der Rahmen (5) zwei ortsfeste Führungsglieder (6, 7) aufweist, die parallel zueinander und zur Grundplatte angeordnet sind (1);
- zwei Schlitten (8), wobei einer der beiden Schlitten (8) an jedem der beiden ortsfesten Führungselemente (6, 7) angeordnet ist und in der Lage ist, entlang des jeweiligen ortsfesten Führungselements (6, 7) in eine Richtung zu gleiten Senkrecht zur Längsachse der Grundplatte (1), wobei jeder Schlitten (8) einen anderen umgekehrten Schlitten mit einem beweglichen Führungsarm (9) trägt, der in einer Richtung senkrecht zur Bewegungsrichtung des Schlittens gleiten kann (8), so daß die Bewegung parallel zur Längsachse der Grundplatte (1) ist, so daß sich die beiden beweglichen Führungsarme (6, 7) in zwei parallelen Ebenen und parallel zur Grundplatte (1) frei bewegen können;
- ein System mit sphärischen Stützen (10), die eine magnetische Kraft oder ein Absaugen. verwenden, um die sphärischen Stützen (10) an den beweglichen Führungsarmen (9) zu befestigen und in der Lage ist, in der Kraft der Befestigung zwischen den kugelförmigen Stützen (10) unterschiedliche Intensitäten zu erzeugen, Und den beweglichen Führungsarmen (9), wobei die sphärischen Stützen (10) frei drehbar sind, so dass eine durch die Mitten der sphärischen Stützen (10) verlaufende hypothetische Linie jeden beliebigen Weg definieren kann, dem eine Nadel folgen kann 11), wobei jeder kugelförmige Träger (10) ein durchgehendes Durchgangsloch aufweist.

2. Stereotaktische Ganzkörperführung nach Anspruch 1, **dadurch gekennzeichnet, daß** die sphärischen Stützen (10) an einem der Enden jedes der beweglichen Führungsarme (9) befestigt sind, wobei die Position jedes kugelförmigen Trägers (10) Raum wird also durch die Lage der beiden Schlitten (8) an den ortsfesten Führungselementen bestimmt

3. Stereotaktisches Ganzkörperführungssystem nach Anspruch 1, **dadurch gekennzeichnet, dass** es ein System von Bezugszeichen (2) zur entsprechenden Abbildung des Koordinatensystems eines Scanners auf das Koordinatensystem des stereotaktischen Ganzkörperführungssystems aufweist System von Bezugszeichen, die aus einer geometrischen Form bestehen, die aus einem Material besteht, das für den Scanner sichtbar ist, der in die mit dem stereotaktischen Ganzkörperführungssystem verbundene Grundplatte (1) eingebettet ist und auf dem Tisch angeordnet ist, auf dem sich der Patient befindet.

4. Stereotaktische Ganzkörperführung nach Anspruch 1, **dadurch gekennzeichnet**, das sie ein System zum Ausrichten von flexiblen Nadeln aufweist, die aus drei Scheiben (18,19,20) aus einem leichten Material bestehen, wobei die ersten beiden Scheiben (18, 19 ) Durch starre Stangen (21) verbunden sind, die sie parallel zueinander halten, während die dritte Scheibe (20) zwei lange Stäbe (22) aufweist, die durch zwei Durchgangslöcher in den beiden anderen Scheiben (18, 19) hindurchgehen, wobei die Stangen (22) die dritte Scheibe (20) parallel zu den beiden anderen Scheiben (18, 19) halten, unabhängig von dem Abstand, der sie trennt, wobei die drei Scheiben (18, 19, 20) eine Öffnung (24) in der Mitte haben Eine Nadel (11) und eine Nadelverlängerung (27), hindurchtreten und die Nadel (11) oder Nadelverlängerung (27) die drei Scheiben (18,19,20) und die beiden sphärischen Stützen (10) durchquert, wobei die kugelförmige, (10) die beiden miteinander verbundenen Scheiben (18, 19) in der richtigen Ausrichtung halten, während die dritte Scheibe (20) nahe an die Haut des Patienten gebracht wird, um die Spitze von n richtig zu führen Eedle (11).

5. Stereotaktisches Ganzkörperführungssystem y nach Anspruch 1, **dadurch gekennzeichnet, dass** es ein System zur Überwachung der Patientenbewegung während der Prozedur aufweist, die aus einer Laserdiode (36) besteht, die ihren Strahl auf einen mit einem Funk-transparenten Bildschirm (35) Zu der Grundplatte (1) des stereotaktischen Ganzkörperführungssystems, wobei der Schirm (35) Mittel (37) zum Markieren der Position des Strahls und einer an die zur Überwachung verwendeten Laserdiode (39) aufweist ln einem Abstand, um sicherzustellen, dass der Laserstrahl auf die Markierung trifft.

## Revendications

1. Système stéréotaxique de guidage de corps entier pour le positionnement précis d'instruments chirurgicaux à l'intérieur du corps, **caractérisé en ce qu'**'il comprend:
- une plaque de base (1) comportant des fiduciels (2)
- un pont (4)
- des moyens de fixation (3) qui assurent l'immobilité et la reproductibilité de la position du pont (4) par rapport à la plaque de base (1)
- un cadre (5) placé sur ou sur l'une des pièces latérales du pont (4), **caractérisé par** le cadre (5) avec deux éléments de guidage stationnaires (6, 7), parallèles l'un à l'autre et à la plaque de base (1);
- deux chariots (8), chaque chariot (8) été situé sur un des deux éléments de guidage stationnaires (6, 7) et pouvant coulisser le long de l'élément de guidage stationnaire respectif (6, 7) dans une direction perpendiculaire à l'axe long de la plaque de base (1), chaque chariot (8) portant un autre chariot inversé avec un bras de guidage mobile (9) pouvant coulisser dans une direction perpendiculaire à la direction de déplacement du chariot (8), de sorte que le mouvement est parallèle à l'axe long de la plaque de base (1), permettant aux deux bras de guidage mobiles (6, 7) de se déplacer librement dans des plans parallèles entre si et parallèles à la plaque de base (1);
et
- un système comprenant des supports sphériques (10) qui utilise une force magnétique ou une aspiration pour attacher les supports sphériques (10) aux bras de guidage mobiles (9) et est capable de produire des intensités différentes dans la force de fixation entre les supports sphériques (10) et les bras de guidage mobiles (9), dans lequel les supports sphériques (10) peuvent tourner librement, de sorte qu'une ligne hypothétique passant par les centres des supports sphériques (10) peut définir tout trajectoire qui peut être suivi par une aiguille (11); dans lequel chaque support sphérique (10) présente un trou traversant son centre.

2. Guide stéréotaxique de corps entier selon la revendication 1, **caractérisé en ce que** les supports sphériques (10) sont fixés à l'une des extrémités de chaque bras de guidage mobiles (9), la position de chaque support sphérique (10) dans l'espace est donc déterminé par la position des deux chariots (8) sur les éléments de guidage stationnaires (6, 7) perpendiculaires au long axe de la plaque de base (1) et par la position des bras de guidage mobiles (9) parallèlemant à ce même axe.

3. Système de guidage stéréotaxique à corps entier selon la revendication 1, **caractérisé en ce qu'**il comporte un système de fiduciel (2) pour établir une relation entre le système de coordonnées d'un scanner et le système de coordonnées du système de guidage stéréotaxique du corps, le mentionné système de fiduciels étant composé d'une forme géométrique formée par un matériau visible par le scanner intégré dans la plaque de base (1) reliée au système stéréotaxique de guidage du corps entier et placée sur la table sur laquelle le patient repose.

4. Guide stéréotaxique de corps entier selon la revendication 1, **caractérisé en ce qu'**il comporte un système pour aligner des aiguilles flexibles composées de trois disques (18, 19, 20) en matériau légère, les deux premiers disques (18, 19) étant connectés par des tiges rigides (21) qui les maintiennent parallèles l'une à l'autre tandis que le troisième disque (20) comporte deux tiges longues (22) traversant deux trous qui traversent aussi les deux autres disques (18, 19), les tiges (22) maintiennent le troisième disque (20) parallèlement aux deux autres disques (18, 19) indépendamment de la distance les séparant. Les trois disques (18, 19, 20) ont un orifice (24) au centre barré par une aiguille (11) o un prolongateur d'aiguille (27), l'aiguille (11) ou, l'extenseur d'aiguille (27) traverse les trois disques (18, 19, 20) et les deux supports sphériques (10). Les supports sphériques (10) maintiennent les deux disques interconnectés (18, 19) dans l'orientation appropriée, tandis que le troisième disque (20) est situé à proximité de la peau du patient de manière à guider correctement la pointe de la aiguille (11).

5. Système de guidage stéréotaxique à corps entier selon la revendication 1, **caractérisé en ce qu'** il comporte un système de surveillance du mouvement du patient pendant la procédure composée d'une diode laser (36), le faisceaux lumineux est projeté sur un écran radio-transparent (35) attaché a la plaque de base (1) du système stéréotaxique de guidage de corps entier, le dit écran (35) comportant des moyens (37) pour marquer la position du faisceau et d'une caméra USB (39) attachée à la diode laser utilisée pour la surveillance a distance, pour s'assurer que le faisceau laser frappe la position désirée
